# EUROPEAN PATENT SPECIFICATION

(11) **EP 3 262 419 B1**
(45) Date of publication and mention of the grant of the patent: **23.06.2021**
(21) Application number: 16710107.0
(22) Date of filing: 26.02.2016
(51) Int. Cl.: G01N 33/68

(54) **PROGNOSIS IN PATIENTS WITH ACUTE CORONARY SYNDROME UNDERGOING PERCUTANEOUS CORONARY INTERVENTION**
PROGNOSE BEI PATIENTEN MIT AKUTEM KORONARSYNDROM UND PERKUTANEM KORONAREINGRIFF
PRONOSTIC CHEZ DES PATIENTS PRÉSENTANT UN SYNDROME CORONARIEN AIGU SUBISSANT UNE INTERVENTION CORONARIENNE PERCUTANÉE

(30) Priority: 27.02.2015 GB 201503425
(43) Date of publication of application: 03.01.2018
(73) Proprietor: Randox Laboratories Ltd, Crumlin, Antrim BT29 4QY (GB)
(72) Inventor: FITZGERALD, Peter, Crumlin Antrim BT29 4QY (GB); LAMONT, John, Crumlin Antrim BT29 4QY (GB); SIVANANTHAN, Mohan, Leeds Yorkshire LS1 3EX (GB); HALL, Alistair, Leeds Yorkshire LS1 3EX (GB); PEARSON, Ian, Leeds Yorkshire LS1 3EX (GB)
(86) International application number: PCT/EP2016/054165
(87) International publication number: WO 2016/135335

(56) References cited:
- WO-A2-2009/150181
- US-A1- 2007 243 632
- LEVENT OZDEMIR ET AL: "Heart Type Fatty Acid Binding Protein Is More Sensitive Than Troponin I and Creatine Kinase Myocardial Band at Early Stage in Determining Myocardial Injury Caused by Percutaneous Coronary Intervention", INTERNATIONAL HEART JOURNAL, vol. 52, no. 3, 1 January 2011 (2011-01-01), pages 143-145, XP055267222, JP ISSN: 1349-2365, DOI: 10.1536/ihj.52.143
- HAFIDH A ALHADI ET AL: "Validity of Cardiac Markers as Diagnostic and Prognostic Indicators of Complications in Patients undergoing Percutaneous Coronary Intervention", SULTAN QABOOS UNIVERSITY MEDICAL JOURNAL, vol. 10, no. 1, 1 April 2010 (2010-04-01), pages 31-40, XP055267238, Oman

## Description

### Background

Patients with acute coronary syndrome (ACS) who undergo percutaneous coronary intervention (PCI) remain at risk of recurrent ACS up to 1 year following intervention, despite the use of dual antiplatelet therapy with aspirin and clopidogrel. Poor response to clopidogrel is a risk factor for recurrent ACS, yet the majority of poor clopidogrel responders do not experience recurrent events. Indiscriminate use of alternative, more potent antiplatelet agents may lead to increased rates of bleeding. The process of balloon inflation and stent deployment necessarily results in a small degree of injury to the wall of the coronary artery and myocardial ischaemia. This is reflected in minor elevations of two markers of myocardial injury, creatinine kinase and cardiac troponin. Studies have shown that this may occur in approximately one third of patients undergoing coronary angioplasty. The significance of this observation however remains unknown. Heart Fatty acid binding protein (H-FABP) is a small protein (14-15 kDa) released from cardiac myocytes following injury. Detectable levels occur 2-3 hours after myocardial injury and return to baseline within 12-24 hours. In combination with 4th generation troponin assays measuring H-FABP levels has been shown to improve diagnosis of acute coronary syndrome (McCann *et al.,* 2008) and predict prognosis independently of troponin (Kilkullen *et al.,* 2007).

### References

Kilcullen, N, et al. (2007) Journal of the American College of Cardiology; 50:2061-2067.

McCann, C, et al. (2008) European Heart Journal; 29:2843-2850.

### Brief description of the drawings

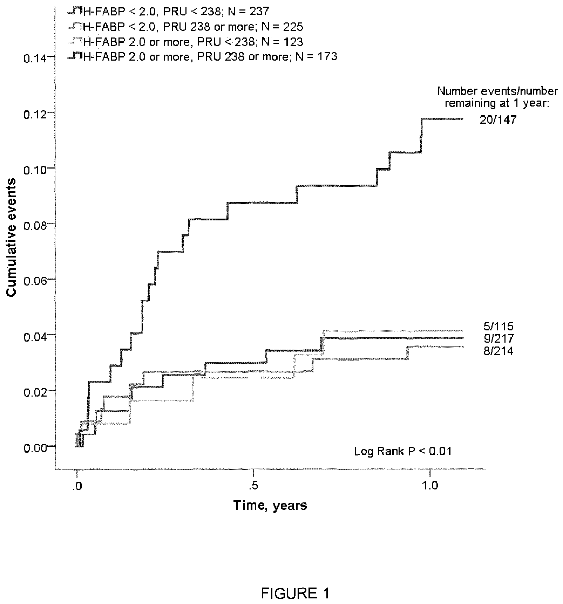

Figure 1 - Cumulative ACS events for patients stratified on the basis of H-FABP levels at baseline following PCI and platelet function tests performed prior to PCI.

### Summary of the Invention

The current invention provides an improved method for more precise prognosis of ACS patients undergoing PCI. The combination of performing a platelet function test prior to PCI and measuring H-FABP prior to PCI can more precisely risk-stratify the patients and has the potential to allow selection of patients who may benefit from more intense platelet inhibition.

### Detailed description of the invention

In a first aspect the current disclosure provides a method for the prognosis of acute coronary syndrome patients (ACS) undergoing percutaneous coronary intervention (PCI), the method comprising; Determining the level of H-FABP in a sample taken from said patient immediately before PCI; Providing a prognosis on the basis of the results of step a) wherein those patients with elevated H-FABP as determined in step a) are at greater risk of future adverse events.

In a further aspect the current invention provides a method for the prognosis of acute coronary syndrome patients (ACS) undergoing percutaneous coronary intervention (PCI), the method comprising; Performing a platelet function test on a sample taken from said patient before PCI; Determining the level of H-FABP in a sample taken from said patient immediately before PCI; Providing a prognosis on the basis of the results of steps a) and b) wherein those subjects with high platelet activity as determined in step a) in combination with elevated H-FABP as determined in step b) are at greater risk of future adverse events.

Acute coronary syndrome (ACS) refers to a set of signs and symptoms usually a combination of chest pain and other features interpreted as being a result of abruptly decreased blood flow to the heart (cardiac ischemia). The subtypes of ACS include unstable angina (UA, not associated with heart muscle damage) and two forms of myocardial infarction (non-ST & ST elevated) in which heart muscle is damaged.

The term 'at greater risk of future adverse events' as used herein refers to patients who are more likely to have a recurrent or subsequent ACS event following the PCI procedure. For the purposes of the current invention this included events that occurred up to one year after the PCI procedure.

The platelet function test can be any suitable test known to those skilled in the art which will give a measurement or indication of high platelet activity in a given sample. High platelet activity as used herein refers to a platelet activity level above a set cut-off or reference level given in the test which is used. The current invention used the VerifyNow test by Accumetrics. In a preferred embodiment of the current invention high platelet activity is defined as a P2Y12 reactivity unit (PRU) value of greater than or equal to 238. Alternatively the skilled person will understand that the particular cut-off value used can be any cut-off value in the prior art which is understood to be indicative of high platelet activity. For example the cut-off value may be greater than or equal to 200 PRU, greater than or equal to 235 PRU or greater than or equal to 240 PRU. Preferably the platelet function test is performed on a sample taken from the patient just prior to the PCI procedure. The term 'just prior' preferably means immediately before the PCI however it will be understood that the platelet function test may be performed on a sample taken a few hours before the PCI. High platelet activity in patients who have been administered an antiplatelet drug indicates that said patients are 'hypo-responders' to that drug. These patients may also be described as 'resistant' to that drug.

The term 'H-FABP' as used herein refers to heart fatty acid-binding protein, also known as fatty acid binding protein 3 (FABP-3), as well as fragments or partial peptides thereof, which in humans is encoded on the *FABP3* gene. H-FABP is present in the myocardium and is believed to be rapidly released into circulation as a result of myocardial injury. Numerous studies have demonstrated that it is an early marker for occurrence of MI. It will be understood to those skilled in the art that H-FABP incorporates any variant polypeptides which share the same essential biological and immunological properties as the specific H-FABP peptides used in the current invention.

Preferably in the methods of the current invention the H-FABP levels are measured in samples taken from the subjects immediately before the PCI procedure (also known as the baseline value). However it will be understood that H-FABP levels measured in samples taken before this may also be useful in the methods of the current invention. This may include samples taken at time points including but not limited to, 1 hour before the procedure, 2 hours before the procedure, 4 hours before the procedure, 6 hours before the procedure and 12 hours before the procedure. While H-FABP levels are preferably measured in samples taken immediately before the PCI procedure those taken after the PCI procedure may also be useful in the methods of the current invention. This may include samples taken at time points including but not limited to, immediately after the procedure, 1 hour after the procedure, 2 hours after the procedure, 4 hours after the procedure, 6 hours after the procedure and 12 hours after the procedure.

The term 'H-FABP level' refers to the concentration of H-FABP determined in the sample taken from the patient, in a preferred embodiment preferably this refers to the concentration in a plasma or serum sample from a subject of the current invention. The 'reference values' or 'cut-off levels' used in the current invention can be commonly acceptable levels of H-FABP which are indicative of ACS. For the purposes of the current invention any value less than these reference levels may be referred to as a 'negative' test, while any value greater than these reference levels may be referred to as a 'positive' test. For the purposes of the current invention the reference value for H-FABP was 2.0 ng/ml. Any H-FABP value below this value is considered a negative result and indicates that the patient is at lower risk of a future ACS event. Those skilled in the art will recognise that alternative H-FABP cut-off values could also be used. For example the H-FABP reference value could be based on the 95th percentile of a reference population as determined by the manufacturer.

A 'subject' or 'patient' of the current invention is preferably a human subject, most preferably one whom presents with ACS without cardiogenic shock and whom is undergoing a planned PCI. The subjects of the current invention are preferably not those undergoing primary PCI although the methods of the current invention may also have utility in this population following the results of future studies. Primary PCI refers to patients admitted to hospital and passed immediately to the cath lab for intervention. In one preferred embodiment the patients undergoing H-FABP testing would be patients who have been deemed to be hypo-responders to an antiplatelet drug or treatment. The patients of the current invention underwent PCI on average around 6-7 days after admission to the hospital. During this period the majority of patients received the antiplatelet drug Clopidogrel.

The sample from said subjects of the current invention can be selected from a body fluid, separated cells or tissue sample. Most preferably the sample is a body fluid, even more preferably the sample is serum or plasma. In a preferred embodiment of the current invention the *in vitro* determination of H-FABP is carried out by immunoassay using any means known in the art. In another preferred embodiment the immunoassay is incorporated into an automated analyser device.

A further embodiment of the current invention is a method of treatment of patients deemed at greater risk following the application of the prognostic methods described above. Preferably these patients will include those who have been found to have elevated H-FABP prior to the PCI. Said treatment, for example, may be further or additional antiplatelet treatments or a longer duration of antiplatelet treatment.

### Methods

We studied the combined endpoint of all cause death, definite or probable stent thrombosis and recurrent myocardial infarction (MI) at 1 year in 758 patients presenting with ACS without cardiogenic shock and undergoing PCI. Patients undergoing primary PCI were not included in this analysis. Cardiac biomarkers were measured at baseline, 4 and 12 hours following PCI.

### Biomarker analysis

Prior to analysis, aliquots were thawed on a mechanised roller for at least 20 minutes at room temperature. Once biomarker analysis had been performed, any residual sample aliquot was discarded. Biomarker analysis was only performed on samples having undergone 1 freeze thaw cycle. H-FABP concentration was measured using a cardiac biochip according to the manufacturer's instructions for use (Randox Laboratories, Crumlin, Northern Ireland). This is a solid state commercially available method based on the two step sandwich chemiluminescent immunoassay principle.

The biochip assays were performed as follows:
1. Two vials of calibrator and control reconstitution materials were reconstituted with double distilled water and placed on a mechanised roller for 10 minutes to ensure adequate mixing.
2. Nine vials of lypophilised calibrator material and 3 vials of lypophilised control material were reconstituted with the solution from step one. Once reconstituted these vials were placed on a mechanised roller for 10 minutes.
3. 240 µl of 19mMol tris-(hydroxymethyl)-aminomethane (TRIS) buffered saline, pH 7.0 containing protein surfactant, preservatives and blocking agents was pipetted onto each of the nine wells of the cardiac biochip array. Micro-pipetted on to this was 60 µl of sample,calibrator or control material. The biochips were then placed onto a thermoshaker device, and incubated at 37°C for 30 minutes at 380 rpm.
4. Following incubation, biochips were emptied of their reagent mixture. Adding approximately 350 µl TRIS buffered saline, pH 7.4, to each sample well, two quick washes and 4 x 2 minute soaks were performed.
5. Following the wash cycles, biochips were tapped dry of surface wash solution on lint free paper.
6. 300 µl of 19mM TRIS buffered saline, pH 7.0 with protein surfactant, stabilizers, preservatives, blocking agents and assay specific horseradish peroxidise (HRP) was then pipetted into each sample well. This was placed on the thermoshaker and incubated at 37°C for 30 minutes at 380 rpm.
7. Following incubation a further wash cycle identical to the process in step 4 was performed.
8. To prevent sample wells drying buffered wash solution was left in each well until analysis.
9. The biochip was tapped dry on lint free paper. 250 µl of signal reagent (1:1 solution luminol and peroxide) was then added to each well and the biochip placed into the carrier of the Evidence Investigator imaging machine exactly 2 minutes +/-10 seconds after the addition of signal reagent. The Evidence Investigator takes two high resolution pictures of the biochip. Automated quantification of sample fluorescence (and hence biomarker concentration) is then performed. In cases of software failure, images can be manually analysed.

A platelet function test was performed according to manufacturors instructions just prior to PCI (VerifyNow, Accumetrics, California, USA). Procedural technique, adjunctive pharmacology and stent type were chosen by the operator.

### Follow up

Post discharge patients were followed up by telephone at 3, 6 and 12 months. At each follow up interview a questionnaire was completed and verification of events confirmed by reference to medical notes.

### End Points

Occurrence of any Major Adverse Coronary Event defined as;
Death (all cause)
Death due to cardiovascular event (MI, arrhythmia and heart failure)
Q wave myocardial infarction
Non Q wave myocardial infarction
Troponin positive (>0.03) acute coronary syndrome
Stroke
Admission with heart failure
Admission with ventricular arrhythmia
Admission with unstable angina
Repeat angiography
Repeat angioplasty (target lesion or de novo lesions)
CABG

### Results

111 presented with unstable angina, 536 with non-STEMI and 111 with STEMI (convalescent PCI). Mean age: 62 (12) years; 25% female; median SYNTAX score 8.0 (11.0). Clopidogrel was given to all but 3 patients; 97% were loaded > 24 hours prior to PCI. 97% received aspirin, 28% IIbIIIa inhibitors. Drug eluting stents were used in 57%.

Clopidogrel hypo-response (PRU ≥ 238) was found in 52.8%. H-FABP was elevated (≥ 2.0 µg/L) at baseline in 40%; of these, H-FABP was also elevated in 90% and 93% at 4 and 12 hours.

Clopidogrel hypo-responders who also had elevated H-FABP levels at baseline (173 patients, 22.8%) had a higher rate of the combined adverse event endpoint at 1 year, whereas hypo-responders with normal H-FABP levels at baseline had a similar event rate to clopidogrel responders; adjusted hazard ratio (95% C.I.) 2.78 (1.34 to 5.69), p < 0.01 (see Figure 1).

The other biomarkers tested (highly sensitive Troponin I, CK-MB and myoglobin) provided little additional prognostic information.

### Conclusion

Combining routine H-FABP testing with platelet function testing can more precisely risk-stratify ACS patients undergoing PCI. This approach has the potential to allow selection of patients who may benefit from more intense platelet inhibition.

## Claims

1. A Method for the prognosis of acute coronary syndrome patients (ACS) undergoing percutaneous coronary intervention (PCI), the method comprising;
a) Performing a platelet function test on a sample that has been taken from said patient before PCI;
b) Determining the level of H-FABP in a sample that has been taken from said patient before PCI;
c) Providing a prognosis on the basis of the results of steps a) and b) wherein those patients with high platelet activity as determined in step a) in combination with elevated H-FABP as determined in step b) are at greater risk of future adverse events.

2. The method of claim 1 wherein said ACS patients are clopidogrel-resistant.

3. The method of any preceding claim wherein the sample is whole blood, serum or plasma.

## Patentansprüche

1. Ein Verfahren zur Prognose von Patienten mit akutem Koronarsyndrom (ACS), die sich einer perkutanen Koronarintervention (PCI) unterziehen, wobei das Verfahren umfasst:
a) Durchführen eines Thrombozytenfunktionstests an einer Probe, die dem Patienten vor der PCI entnommen wurde;
b) Bestimmen des H-FABP-Spiegels in einer Probe, die dem Patienten vor der PCI entnommen wurde;
c) Bereitstellen einer Prognose auf der Grundlage der Ergebnisse der Schritte a) und b), wobei diejenigen Patienten mit hoher Blutplättchenaktivität, wie in Schritt a) in Kombination mit erhöhtem H-FABP, wie in Schritt b) bestimmt, bestimmt sind, einem höheren Risiko von zukünftige unerwünschte Ereignisse.

2. Verfahren nach Anspruch 1, wobei die ACS-Patienten Clopidogrel-resistent sind.

3. Verfahren nach einem vorhergehenden Anspruch, wobei die Probe Vollblut, Serum oder Plasma ist.

## Revendications

1. Une méthode pour le pronostic des patients atteints du syndrome coronarien aigu (SCA) subissant une intervention coronarienne percutanée (PCI), la méthode comprenant;
a) réalisation d'un test de fonction plaquettaire sur un échantillon qui a été prélevé sur ledit patient avant l'ICP;
b) déterminer le niveau de H-FABP dans un échantillon qui a été prélevé sur ledit patient avant l'ICP;
c) fournir un pronostic sur la base des résultats des étapes a) et b) où les patients ayant une activité plaquettaire élevée telle que déterminée à l'étape a) en combinaison avec une H-FABP élevée telle que déterminée à l'étape b) courent un plus grand risque d'avenir événements indésirables.

2. Procédé selon la revendication 1 dans lequel lesdits patients SCA sont résistants au clopidogrel.

3. Procédé selon l'une quelconque des revendications précédentes dans lequel l'échantillon est du sang total, du sérum ou du plasma.
